Europäisches Patentamt

European Patent Office

Office européen des brevets

(11) Publication number: **0 066 374**
**B1**

## (12) EUROPEAN PATENT SPECIFICATION

(45) Date of publication of patent specification: **25.09.85**

(21) Application number: **82302276.9**

(22) Date of filing: **05.05.82**

(51) Int. Cl.⁴: **A 61 B 1/00, G 02 B 7/04**

(54) **Endoscopes.**

(30) Priority: **29.05.81 JP 82389/81**

(43) Date of publication of application:
**08.12.82 Bulletin 82/49**

(45) Publication of the grant of the patent:
**25.09.85 Bulletin 85/39**

(84) Designated Contracting States:
**AT BE CH DE FR GB IT LI NL SE**

(56) References cited:
**EP-A-0 027 651**
**DE-C- 408 969**
**US-A- 315 828**
**US-A-3 132 646**
**US-A-3 178 994**
**US-A-3 261 349**
**US-A-4 163 148**

(73) Proprietor: **OLYMPUS OPTICAL CO., LTD.**
**43-2, 2-chome, Hatagaya Shibuya-ku**
**Tokyo 151 (JP)**

(72) Inventor: **Taguchi, Akihiro**
**No. 16-4, Myojincho 1-chome Hachioji-shi**
**Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

## Description

This invention relates to endoscopes of the hard type containing an objective system, together with a relay lens system for transmitting an optical image of an object formed by the objective system.

In a conventional endoscope that is to be inserted into a body cavity or the like for observation, diagnosis or treatment by using forceps, an image is formed by an objective system arranged at the top of an inserted part, and this optical image is transmitted to an eyepiece system arranged in the rear operating part by a relay lens system so that this transmitted optical image of the object can be observed by an operator with the naked eye, recorded by a photographic camera or viewed via a television camera fitted to the rear part of the endoscope.

Such endoscopes are often used in cases where an image of an affected part must be clear in detail, to permit magnified observation, particularly to assist diagnosis of the affected part.

As a means of coping with such case, there is suggested in the Gazette of Japanese Utility Model Laid Open No. 87180/1977 a construction having its objective system arranged movable in the axial direction inside the tip of an inserted part of the endoscope, a pulley fitted near this lens system and a control wire extended out of the tip of the inserted part to engage the above-mentioned lens system through this pulley from an operating part so as to move the lens system forward rearward. However, in this conventional formation, as the lens system is moved in both forward and rearward directions with the drawing wire, there are problems that a pulley is required for the wire at the end of the inserted part, there is a lower limit for the diameter of the wire, a pipe is required to cover this drawing wire, and therefore the disadvantage arises that the outside diameter of the inserted part of the endoscope, which is naturally required to be made as small as possible to reduce the pain of the patient and to improve the inserting operability, becomes inevitably large and the structure complicated in this known formation. Further, in this prior art formation, if only one control wire is used, it is not possible to ensure that the lens system will be able to be stably moved and, even if a plurality of wires are used, the tensions acting on the respective wires will be difficult to maintain uniform and it may still be difficult to move the lens system in a stable manner.

Further, there is suggested an endoscope wherein, while an objective system is arranged movably in the axial direction inside the tip of an inserting part, a coil spring and electromagnet are incorporated for forward and rearward movement of this lens so that the lens may be moved forward and rearward by the repulsion and attraction of this electromagnet and the energising force of the coil spring. However, in such formation, it is difficult to properly adjust the repulsion and attraction of the electromagnet and the energising force of the coil spring to ensure positive stabilised movement of the lens.

The United States Patent No. 4 149 769 and West German Laid Open Patent Publication No. 26 40 353 describe an endoscope wherein a substantially tubular elongate jacket having one objective system arranged at the front end and one eyepiece system arranged at the rear end is so provided that an inserted part of an optical sighting tube can be inserted into this jacket.

The above-mentioned optical sighting tube is formed to be movable forward and rearward within the jacket tube along a longitudinal groove formed on the rear end side of the jacket so that, when the optical sighting tube is moved, an image of an object will be varied in magnification by the two objective systems on being transmitted through relay lens systems arranged within the optical sighting tube to be observed in the eyepiece system.

In this prior art, there are problems that, when the optical sighting tube is moved forward and rearward, an image will be able to be formed and varied in magnification by the objective systems at the front end of the jacket tube and at the tip of the optical sighting tube but, in this case, the eyepiece systems will also move together and therefore, though a small variation of the magnification may be possible, a comparatively large variation of magnification (particularly enlargement), such as may be achieved in a zooming optical system will not be possible. Further, most of the intermediate lens arrangements are not changed at all, but only relative movement of the front and rear lenses to obtain a desired performance and the design of the lenses is more restricted than in the present invention, wherein any of the intermediate lens systems may be moved. The means of moving the optical sighting tube forward and rearward (with respect to the jacket) for the observation as varied in the magnification in the above is not provided with a fine movement adjusting means and, in the case of a clear observation in detail, the movable operation directly moving the optical sighting tube forward and rearward with respect to the jacket will be difficult and inconvenient.

Similar optical problems are encountered in a very different art, where questions of safety and hygiene do not arise, and the United States Patent No. 3 178 994 describes a borescope having means to zoom or vary magnification by mounting a field lens and associated relay lenses in an inner tube mechanically coupled to an outer zoom control that is manually slidable near the eyepiece, being arranged to permit rotation of an intermediately positioned tube between the inner tube and outer housing in order to provide positional control of a mirror mounted on a pivot to vary the angle of view with respect to the optical axis. Intense illumination, and associated heat, is produced by a quartz iodine lamp mounted at the forward end, and fed via the housing and intermediate tube, which are insulated from each other, and therefore the device is quite unsuited for use as a diagnostic medical endoscope, because of the heat, the unsealed housing and the exposed electrical connections. However, the optical system from the eyepiece to the fixed objective lens can be considered alone, and provides, in combination, a fixed objective lens at the end of a tube that extends to an eyepiece and contains a plurality of relay lenses set at fixed spacings by spacer tubes, and an inner tube carrying a field lens and further relay lens spaced by respective spacer tubes, and all slidable within the tube that carries the objective lens, to provide a zoom action.

One object of the present invention is to provide an endoscope wherein the magnification may be varied substantially without enlarging the diameter of an inserted part, while enabling details to be clearly observed, and permitting fine adjustment to be easily made, both of magnification and of focus.

Another object of the present invention is to realise the above-mentioned precision and flexibility with a relatively simple structure of low cost.

According to the present invention there is provided an endoscope having an observing optical system wherein an image of an object is front of an inserted part is formed by an objective system arranged within a lens tube inserted through an inner tube within the inserted part and the formed optical image is transmitted rearward by a relay lens systems arranged within the lens tube to the rear of said objective system so as to be observed from the rear of an operating part, and means being provided to move a lens system that forms part of said objective system or said relay lens systems to make magnification variation or focus adjustment possible, characterised in that: the movable lens system is connected to the tip of a sliding pipe arranged between the lens tube and inner tube and the sliding pipe is coupled within the operating part to a driving mechanism provided in the operating part and having a fine movement adjusting function; that the lens tube is fixed to the inner tube at its forward tip and extends from the objective system to the operating part where it carries an eyepiece system; that the inner tube forms an inner wall of an annular gap within an outer housing of the insertion part, which gap contains light guide fibres; and that an external control on the operating part is provided for the driving mechanism.

The invention will now be described with reference to the drawings, in which:—

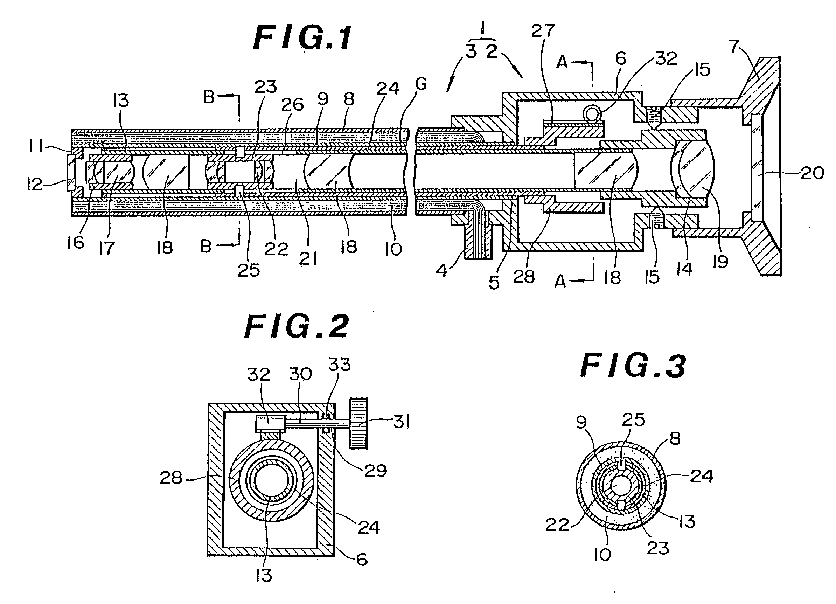

Figure 1 is a vertical section showing details of one exemplary embodiment of an endoscope constructed in accordance with the present invention;

Figure 2 is a section on line A—A in Figure 1; and

Figure 3 is a section on the line B—B in Figure 1.

In the endoscope embodiment illustrated an endoscope 1 comprises an operating part 2 and insertion part 3. The operating part 2 is formed by a holding frame 6 having a connector 4 on the outer periphery, a flange 5 projecting inwardly from the internal wall, and an eyepiece frame 7, joined together by screwing, bonding or fusing. On the other hand, the inserted part 3 is formed of an outer tube 8 fitted and fused into the front of the operating part 2 and projecting therefrom, an inner tube 9 inserted into the operating part 2 from the tip of the outer tube 8 and fitted and fused or bonded in the rear part to the flange 5 of the holding frame 6 and light guide fibres 10 arranged in the annular space between the outer tube 8 and inner tube 9 and extended out in the rear part to the end surface of the connector 4 of the holding frame 6 and to the end surface of the outer tube 8 at the front.

A cover glass 12 is fitted and bonded through a frame 11 to the tip of the inner tube 9, a lens tube 13 is inserted and arranged in the operating part 2 from the rear of the cover glass 12 with a gap G left within the inner tube 9. This lens tube 13 is fused on the front outer periphery to the inner tube 9, is projected in the rear part into the operating part 2 from the rear end of the inner tube 9 and has a supporting tube 14 secured to this projected part. This supporting tube 14 is held by screws 15 screwed in from the outer periphery of the holding frame 6 to support the lens tube 13 in the rear part. Within the lens tube 13, an objective system 17 is arranged and fixed through a frame 16, and relay lens systems 18 are arranged and fixed in the rear of it. Also, an eyepiece system 19 is fitted and bonded in the supporting tube 14 in the rear of the lens tube 13, and a cover glass 20 is fitted and bonded in a rear opening of the eyepiece frame 7.

A hollow cavity 21 is formed within the lens tube 13 between the relay lens system 18 adjacent to the objective system 17 and the relay lens system 18 and a sliding lens frame 23 fitted with a magnification varying lens system 22 is arranged slidably in the axial direction within this hollow cavity 21. Further, a sliding pipe 24 is inserted and arranged in the gap G between the above mentioned inner tube 9 and lens tube 13 and has pins 25 projecting on the inner periphery at the front end. These pins 25 are locked and fixed in the sliding lens frame 23, through axial slits 26 formed in the lens tube 13, to connect the sliding pipe 24 and sliding lens frame 23 with each other. The sliding lens frame 23 is thus slidable forward and rearward by the length of the slits 26 of the lens tube 13 within the hollow cavity 21. The above mentioned sliding pipe 24 projects at the rear into the operation part 2, and a rack fitting member 28 having a rack 27 is secured to this projecting part. The holding frame 6 has a through-bore 29, through which a shaft 30 is inserted and supported within the operating part 2. This shaft 30 has an operating knob 31 secured to the outer end and a pinion 32 secured to the inner end, and this pinion 32 is meshed with the rack 27. The through-bore 29 has a packing seal 33 arranged in it, to make the casing watertight. It is one of the features that the pinion 32 is so formed that, when the inclination of the teeth meshing with the rack 27 is large, that is, the forward or rearward movement of the rack 27 when the shaft 30 of the pinion 32 makes one rotation is small, so that the finely adjusting function in the case of the forward and rearward movement of the magnification varying lens system 22 will be retained.

Further it is another feature of this embodiment that, as the only movable part 22 of the optical system forms a part of the objective system 17 or the relay lens system 18 is movable, the load on the movable part can be made small, the sliding pipe 24 can be made light and thin, and it is not necessary to substantially enlarge the diameter of the inserted part 3.

In the above-mentioned case, if the magnification varying lens system 22 is provided on the relay lens system 18 side, the sliding pipe 24 will be able to be made short, giving advantages in weight reduction and manufacture.

In observation, varied magnification is obtained when the operating knob 31 is rotated and operated, the pinion 32 will be rotated, the rack 27 meshing with this pinion 32 will be moved forward or rearward, and this movement will be transmitted to the sliding lens frame 23 through the sliding pipe 24, so that the magnification varying lens system 22 will be moved forward or rearward and thereby the magnification will be able to be varied from a small value to a large value.

The magnification varying lens system 22 in this case uses a zoom optical system. If a lens system which is not purely a zoom optical system is used, the magnification varying lens system 22 can be used to adjust the sight or focus.

In the above-described embodiment of the present invention, the movable lens system 22 forms a part of the relay lens systems 18. However, it may be so formed that the magnification can be varied or the focus can be adjusted by making the moving system act as a part of the objective system itself. Further, the driving mechanism for sliding the sliding pipe 24 need not be formed by a rack and pinion, but also other known mechanisms, for example, a cam means wherein a longitudinal groove is formed in the outer tube in the operating part (or an extension of the outer tube to the operating part or a substantial equivalent or, in some cases, the lens tube), a pin provided to project on the outside from the inside sliding pipe 24 is passed through the longitudinal groove and further a ring having a cam groove formed to contain this pin is externally fitted to the above-mentioned outer tube so as to be rotatable in the peripheral direction. In such case, the ring is secured so as not to move in the longitudinal direction.

According to the above-mentioned formation, when the ring is rotated, the pin guided and regulated by the cam groove will move the sliding pipe 24 forward and rearward. In such case, if the cam groove is made close to a lateral groove, that is to say, is made small in the distance through which the pin moves in the axial direction (longitudinal direction) when rotating the outer periphery of the ring, the fine movement adjusting function will be retained and the magnification variation or focus adjustment will be able to be easily made.

If a sliding pipe provided on the side surface with a groove, hole or opening of any other shape is used, the overall weight may be reduced.

## Claims

1. An endoscope having an observing optical system wherein an image of an object in front of an inserted part (3) is formed by an objective system (17) arranged within the lens tube (13) inserted through an inner tube (9) within the inserted part and the formed optical image is transmitted rearward by a relay lens systems (18) arranged within the lens tube to the rear of said objective system so as to be observed from the rear of an operating part (2), and means being provided to move a lens system (22) that forms part of said objective system or said relay lens systems to make magnification variation or focus adjustment possible, characterised in that:

the movable lens system (22) is connected to the tip of a sliding pipe (24) arranged between the lens tube (13) and inner tube (9) and the sliding pipe is coupled within the operating part to a driving mechanism (32) provided in the operating part (2) and having a fine movement adjusting function;

that the lens tube (13) is fixed to the inner tube (9) at its forward tip and extends from the objective system (17) to the operating part where it carries an eyepiece system (19);

that the inner tube (9) forms an inner wall of an annular gap within an outer housing (8) of the insertion part (3), which gap contains light guide fibres (10); and

that an external control (31) on the operating part (2) is provided for the driving mechanism (32).

2. An endoscope as claimed in Claim 1, characterised in that the driving mechanism (32) comprises a pinion connected to the rear end of the sliding pipe (24) and a rack (27) meshing with the pinion.

3. An endoscope as claimed in Claim 1, characterised in that the driving mechanism (32) comprises a pin (25) projecting on the rear end of the sliding pipe (24) through a longitudinal groove provided in the lens tube (13) and contained at the tip in a rotatable ring in which a cam groove is formed.

## Revendications

1. Endoscope muni d'un système optique d'observation dans lequel l'image d'un objet venant en face d'une partie insérée (3), est formée par un système d'objectif (17) monté à l'intérieur d'un tube à lentilles (13) introduit par un tube intérieur (9) dans la partie insérée, et dans lequel l'image optique formée est transmise vers l'arrière par un système de lentille de relais (18) monté dans le tube à lentilles en arrière du système d'objectif, de manière à pouvoir être observée par l'arrière d'une partie de manoeuvre (2), et des moyens prévus pour déplacer un système de lentilles (22) faisant partie du système d'objectif ou du système de lentille de relais, pour permettre une variation de grossissement ou un réglage de focalisation, endoscope caractérisé en ce que:

le système mobile (22) de lentilles est relié au bout d'un tube de glissement (24) monté entre le tube à lentilles (13) et le tube intérieur (9), et le tube de glissement est couplé, à l'intérieur de la partie de manoeuvre, à un mécanisme d'entrainement (32) prévu dans la partie de manoeuvre (2), et comportant une fonction de réglage fin du mouvement;

en ce que le tube à lentilles (13) est fixé au tube intérieur (9) à son extrémité avant et va du système d'objectif (17) à la partie de manoeuvre où il porte un système d'oculaire (19);

en ce que le tube intérieur (9) forme la paroi intérieure d'un espace annulaire situé à l'intérieur d'une enveloppe extérieure (8) de la partie d'insertion (3), cet espace annulaire contenant les fibres de guidage de lumière (10); et

en ce qu'une commande extérieure (31) est prévue sur la partie de manoeuvre (2) pour le mécanisme d'entrainement (32).

2. Endoscope selon la revendication 1, caractérisé en ce que le mécanisme d'entrainement (32) comprend un pignon relié à l'extrémité arrière du tube de glissement (24) et une crémaillère (27) venant en prise avec ce pignon.

3. Endoscope selon la revendication 1, caractérisé en ce que le mécanisme d'entrainement (32) comprend une tige (25) faisant saillie sur l'extrémité arrière du tube de glissement (24), à travers une rainure longitudinale prévue dans le tube à lentilles (13) et logée au bout dans un anneau rotatif dans lequel est formée une rainure de came.

**Patentansprüche**

1. Endoskop mit einer Beobachtungsoptik, wobei ein Bild eines Objekts vor einem eingeführten Teil (3) durch ein Objektivsystem (17) gebildet wird, welches im Innern einer Linsenröhre (13), die durch eine Innenröhre (9) innerhalb des eingeführten Teils gesteckt wird, vorgesehen ist, und das erzeugte optische Bild wird rückwärts von einem innerhalb der Linsenröhre vorgesehenen Relaislinsensystem (18) derart zum hinteren Teil des genannten Objektivsystems übertragen, daß es vom hinteren Ende eine Bedienungsteils (2) aus betrachtet wird, und wobei Mittel zum Bewegen eines Linsensystems (22) vorgesehen sind, welches einen Teil des gesamten Objektivsystems oder Relaislinsensystems darstellt, so daß eine Vergrößerungsvariation oder Fokussiereinstellung ermöglicht wird, dadurch gekennzeichnet, daß:

das bewegliche Linsensystem (22) mit dem Ende eines zwischen Linsenröhre (13) und Innenröhre (9) vorgesehenen Gleitrohrs (24) verbunden ist, und das Gleitrohr ist im Innern des Bedienungsteils mit einem Antriebswerk (22) gekoppelt, das im Bedienungsteil (2) vorgesehen ist und eine Bewegungsfeineinstellungsfunktion hat;

daß die Linsenröhre (13) am vordersten Ende der Innenröhre (9) fixiert ist und sich vom Objektivsystem (17) bis zum Bedienungsteil erstreckt, wo sie ein Okularsystem (19) trägt;

daß die Innenröhre (9) eine Innenwand eines ringförmigen, Lichtleiterfasern enthaltenden Zwischenraums (10) innerhalb eines äußeren Gehäuses (8) des eingeführten Teils (3) bildet; und daß für Antriebswerk (32) am Bedienungsteil (2) eine externe Kontrolle (31) vorgesehen ist.

2. Endoskop nach Anspruch 1, dadurch gekennzeichnet, daß das Antriebswerk (32) einen mit dem hinteren Ende des Gleitrohrs (24) verbundenen Ritzel und eine mit dem Ritzel in Eingriff stehende Zahnstange (27) enthält.

3. Endoskop nach Anspruch 1, dadurch gekennzeichnet, daß das Antriebswerk (32) einen Stift (25) enthält, der am hinteren Ende des Gleitrohrs (24) durch eine Längsvertiefung hindurchragt, die an der Linsenröhre (13) vorgesehen und am Ende in einem drehbaren Ring, in welchem eine Nockenvertiefung gebildet ist, enthalten ist.

FIG.1

FIG.2

FIG.3

0 066 374